# EUROPEAN PATENT APPLICATION

(11) **EP 1 362 914 A2**
(43) Date of publication of application: **19.11.2003**
(21) Application number: 03010838.5
(22) Date of filing: 14.05.2003
(51) Int. Cl.: C12N 15/11, A61K 38/00, A61K 48/00, G01N 33/53

(54) **Histone deacetylase inhibitor and use thereof**

(30) Priority: 15.05.2002 JP 2002140586
(71) Applicant: Schering AG, 13353 Berlin (DE)
(72) Inventor: Nakanishi, Osamu, Mobara-shi, Chiba 297-0017 (JP); Tatamiya, Takayuki, Chiba-shi, Chiba 266-0031 (JP)
(74) Representative: Pohlman, Sandra M.

(57) **Abstract**

To provide a novel apoptosis inducer, and a method of screening an apoptosis inducer. For example, an apoptosis inducer comprising inhibiting HDAC6 such as an antisense oligonucleotide to the gene of histone deacetylase 6 (HDAC6), and anti-cancer agent comprising this apoptosis inducer, and the present invention also provides a method of screening an apoptosis inducer that inhibits HDAC6, and more specifically a method of screening an apoptosis inducer said method comprising the steps of (1) determining whether or not a test substance inhibits histone deacetylase 6 (HDAC6) by using deacetylation of an acetylated substance that can be a substrate for histone deacetylase 6 (HDAC6) or decrease in the expression of histone deacetylase 6 (HDAC6) as an index; and (2) confirming, when inhibition is present, whether it induces apoptosis of the cell in vitro and/or in vivo.

SEQUENCE LISTING

<110> Schering Aktiengesellschaft

<120> Histone deacetylase inhibitor and use thereof

<130> 1023370

<160> 31

<210> 1
<211> 4099
<212> DNA
<213> Homo sapiens

<220>
<221>
<222>
<223> DNA encoding histone deacetylase 6

<400> 1
gggcagtccc ctgaggagcg gggctggttg aaacgctagg ggcgggatct ggcggagtgg 60
aagaaccgcg gcaggggcca agcctcctca act atg acc tca acc ggc cag gat 114
Met Thr Ser Thr Gly Gln Asp
1               5
tcc acc aca acc agg cag cga aga agt agg cag aac ccc cag tcg ccc 162
Ser Thr Thr Thr Arg Gln Arg Arg Ser Arg Gln Asn Pro Gln Ser Pro
        10                  15                  20
cct cag gac tcc agt gtc act tcg aag cga aat att aaa aag gga gcc 210
Pro Gln Asp Ser Ser Val Thr Ser Lys Arg Asn Ile Lys Lys Gly Ala
    25                  30                  35
gtt ccc cgc tct atc ccc aat cta gcg gag gta aag aag aaa ggc aaa 258
Val Pro Arg Ser Ile Pro Asn Leu Ala Glu Val Lys Lys Lys Gly Lys
40                  45                  50                  55

atg aag aag ctc ggc caa gca atg gaa gaa gac cta atc gtg gga ctg     306
Met Lys Lys Leu Gly Gln Ala Met Glu Glu Asp Leu Ile Val Gly Leu
                60                  65                  70
caa ggg atg gat ctg aac ctt gag gct gaa gca ctg gct ggc act ggc     354
Gln Gly Met Asp Leu Asn Leu Glu Ala Glu Ala Leu Ala Gly Thr Gly
            75                  80                  85
ttg gtg ttg gat gag cag tta aat gaa ttc cat tgc ctc tgg gat gac     402
Leu Val Leu Asp Glu Gln Leu Asn Glu Phe His Cys Leu Trp Asp Asp
        90                  95                  100
agc ttc ccg gaa ggc cct gag cgg ctc cat gcc atc aag gag caa ctg     450
Ser Phe Pro Glu Gly Pro Glu Arg Leu His Ala Ile Lys Glu Gln Leu
    105                 110                 115
atc cag gag ggc ctc cta gat cgc tgc gtg tcc ttt cag gcc cgg ttt     498
Ile Gln Glu Gly Leu Leu Asp Arg Cys Val Ser Phe Gln Ala Arg Phe
120                 125                 130                 135
gct gaa aag gaa gag ctg atg ttg gtt cac agc cta gaa tat att gat     546
Ala Glu Lys Glu Glu Leu Met Leu Val His Ser Leu Glu Tyr Ile Asp
                140                 145                 150
ctg atg gaa aca acc cag tac atg aat gag gga gaa ctc cgt gtc cta     594
Leu Met Glu Thr Thr Gln Tyr Met Asn Glu Gly Glu Leu Arg Val Leu
            155                 160                 165
gca gac acc tac gac tca gtt tat ctg cat ccg aac tca tac tcc tgt     642
Ala Asp Thr Tyr Asp Ser Val Tyr Leu His Pro Asn Ser Tyr Ser Cys
        170                 175                 180
gcc tgc ctg gcc tca ggc tct gtc ctc agg ctg gtg gat gcg gtc ctg     690
Ala Cys Leu Ala Ser Gly Ser Val Leu Arg Leu Val Asp Ala Val Leu
    185                 190                 195
ggg gct gag atc cgg aat ggc atg gcc atc att agg cct cct gga cat     738
Gly Ala Glu Ile Arg Asn Gly Met Ala Ile Ile Arg Pro Pro Gly His
200                 205                 210                 215
cac gcc cag cac agt ctt atg gat ggc tat tgc atg ttc aac cac gtg     786
His Ala Gln His Ser Leu Met Asp Gly Tyr Cys Met Phe Asn His Val
                220                 225                 230
gct gtg gca gcc cgc tat gct caa cag aaa cac cgc atc cgg agg gtc     834
Ala Val Ala Ala Arg Tyr Ala Gln Gln Lys His Arg Ile Arg Arg Val
            235                 240                 245
ctt atc gta gat tgg gat gtg cac cac ggt caa gga aca cag ttc acc     882
Leu Ile Val Asp Trp Asp Val His His Gly Gln Gly Thr Gln Phe Thr
        250                 255                 260

ttc gac cag gac ccc agt gtc ctc tat ttc tcc atc cac cgc tac gag 930
Phe Asp Gln Asp Pro Ser Val Leu Tyr Phe Ser Ile His Arg Tyr Glu
    265                 270                 275
cag ggt agg ttc tgg ccc cac ctg aag gcc tct aac tgg tcc acc aca 978
Gln Gly Arg Phe Trp Pro His Leu Lys Ala Ser Asn Trp Ser Thr Thr
280                 285                 290                 295
ggt ttc ggc caa ggc caa gga tat acc atc aat gtg cct tgg aac cag 1026
Gly Phe Gly Gln Gly Gln Gly Tyr Thr Ile Asn Val Pro Trp Asn Gln
                300                 305                 310
gtg ggg atg cgg gat gct gac tac att gct gct ttc ctg cac gtc ctg 1074
Val Gly Met Arg Asp Ala Asp Tyr Ile Ala Ala Phe Leu His Val Leu
            315                 320                 325
ctg cca gtc gcc ctc gag ttc cag cct cag ctg gtc ctg gtg gct gct 1122
Leu Pro Val Ala Leu Glu Phe Gln Pro Gln Leu Val Leu Val Ala Ala
        330                 335                 340
gga ttt gat gcc ctg caa ggg gac ccc aag ggt gag atg gcc gcc act 1170
Gly Phe Asp Ala Leu Gln Gly Asp Pro Lys Gly Glu Met Ala Ala Thr
    345                 350                 355
ccg gca ggg ttc gcc cag cta acc cac ctg ctc atg ggt ctg gca gga 1218
Pro Ala Gly Phe Ala Gln Leu Thr His Leu Leu Met Gly Leu Ala Gly
360                 365                 370                 375
ggc aag ctg atc ctg tct ctg gag ggt ggc tac aac ctc cgc gcc ctg 1266
Gly Lys Leu Ile Leu Ser Leu Glu Gly Gly Tyr Asn Leu Arg Ala Leu
                380                 385                 390
gct gaa ggc gtc agt gct tcg ctc cac acc ctt ctg gga gac cct tgc 1314
Ala Glu Gly Val Ser Ala Ser Leu His Thr Leu Leu Gly Asp Pro Cys
            395                 400                 405
ccc atg ctg gag tca cct ggt gcc ccc tgc cgg agt gcc cag gct tca 1362
Pro Met Leu Glu Ser Pro Gly Ala Pro Cys Arg Ser Ala Gln Ala Ser
        410                 415                 420
gtt tcc tgt gct ctg gaa gcc ctt gag ccc ttc tgg gag gtt ctt gtg 1410
Val Ser Cys Ala Leu Glu Ala Leu Glu Pro Phe Trp Glu Val Leu Val
    425                 430                 435
aga tca act gag acc gtg gag agg gac aac atg gag gag gac aat gta 1458
Arg Ser Thr Glu Thr Val Glu Arg Asp Asn Met Glu Glu Asp Asn Val
440                 445                 450                 455
gag gag agc gag gag gaa gga ccc tgg gag ccc cct gtg ctc cca atc 1506
Glu Glu Ser Glu Glu Glu Gly Pro Trp Glu Pro Pro Val Leu Pro Ile
                460                 465                 470

ctg aca tgg cca gtg cta cag tct cgc aca ggg ctg gtc tat gac caa 1554
Leu Thr Trp Pro Val Leu Gln Ser Arg Thr Gly Leu Val Tyr Asp Gln
            475                 480                 485
aat atg atg aat cac tgc aac ttg tgg gac agc cac cac cct gag gta 1602
Asn Met Met Asn His Cys Asn Leu Trp Asp Ser His His Pro Glu Val
        490                 495                 500
ccc cag cgc atc ttg cgg atc atg tgc cgt ctg gag gag ctg ggc ctt 1650
Pro Gln Arg Ile Leu Arg Ile Met Cys Arg Leu Glu Glu Leu Gly Leu
    505                 510                 515
gcc ggg cgc tgc ctc acc ctg aca ccg cgc cct gcc aca gag gct gag 1698
Ala Gly Arg Cys Leu Thr Leu Thr Pro Arg Pro Ala Thr Glu Ala Glu
520                 525                 530                 535
ctg ctc acc tgt cac agt gct gag tac gtg ggt cat ctc cgg gcc aca 1746
Leu Leu Thr Cys His Ser Ala Glu Tyr Val Gly His Leu Arg Ala Thr
                540                 545                 550
gag aaa atg aaa acc cgg gag ctg cac cgt gag agt tcc aac ttt gac 1794
Glu Lys Met Lys Thr Arg Glu Leu His Arg Glu Ser Ser Asn Phe Asp
            555                 560                 565
tcc atc tat atc tgc ccc agt acc ttc gcc tgt gca cag ctt gcc act 1842
Ser Ile Tyr Ile Cys Pro Ser Thr Phe Ala Cys Ala Gln Leu Ala Thr
        570                 575                 580
ggc gct gcc tgc cgc ctg gtg gag gct gtg ctc tca gga gag gtt ctg 1890
Gly Ala Ala Cys Arg Leu Val Glu Ala Val Leu Ser Gly Glu Val Leu
    585                 590                 595
aat ggt gct gct gtg gtg cgt ccc cca gga cac cac gca gag cag gat 1938
Asn Gly Ala Ala Val Val Arg Pro Pro Gly His His Ala Glu Gln Asp
600                 605                 610                 615
gca gct tgc ggt ttt tgc ttt ttc aac tct gtg gct gtg gct gct cgc 1986
Ala Ala Cys Gly Phe Cys Phe Phe Asn Ser Val Ala Val Ala Ala Arg
                620                 625                 630
cat gcc cag act atc agt ggg cat gcc cta cgg atc ctg att gtg gat 2034
His Ala Gln Thr Ile Ser Gly His Ala Leu Arg Ile Leu Ile Val Asp
            635                 640                 645
tgg gat gtc cac cac ggt aat gga act cag cac atg ttt gag gat gac 2082
Trp Asp Val His His Gly Asn Gly Thr Gln His Met Phe Glu Asp Asp
        650                 655                 660
ccc agt gtg cta tat gtg tcc ctg cac cgc tat gat cat ggc acc ttc 2130
Pro Ser Val Leu Tyr Val Ser Leu His Arg Tyr Asp His Gly Thr Phe
    665                 670                 675

ttc ccc atg ggg gat gag ggt gcc agc agc cag atc ggc cgg gct gcg     2178
Phe Pro Met Gly Asp Glu Gly Ala Ser Ser Gln Ile Gly Arg Ala Ala
680                 685                 690                 695

ggc aca ggc ttc acc gtc aac gtg gca tgg aac ggg ccc cgc atg ggt     2226
Gly Thr Gly Phe Thr Val Asn Val Ala Trp Asn Gly Pro Arg Met Gly
                700                 705                 710

gat gct gac tac cta gct gcc tgg cat cgc ctg gtg ctt ccc att gcc     2274
Asp Ala Asp Tyr Leu Ala Ala Trp His Arg Leu Val Leu Pro Ile Ala
            715                 720                 725

tac gag ttt aac cca gaa ctg gtg ctg gtc tca gct ggc ttt gat gct     2322
Tyr Glu Phe Asn Pro Glu Leu Val Leu Val Ser Ala Gly Phe Asp Ala
        730                 735                 740

gca cgg ggg gat ccg ctg ggg ggc tgc cag gtg tca cct gag ggt tat     2370
Ala Arg Gly Asp Pro Leu Gly Gly Cys Gln Val Ser Pro Glu Gly Tyr
    745                 750                 755

gcc cac ctc acc cac ctg ctg atg ggc ctt gcc agt ggc cgc att atc     2418
Ala His Leu Thr His Leu Leu Met Gly Leu Ala Ser Gly Arg Ile Ile
760                 765                 770                 775

ctt atc cta gag ggt ggc tat aac ctg aca tcc atc tca gag tcc atg     2466
Leu Ile Leu Glu Gly Gly Tyr Asn Leu Thr Ser Ile Ser Glu Ser Met
                780                 785                 790

gct gcc tgc act cgc tcc ctc ctt gga gac cca cca ccc ctg ctg acc     2514
Ala Ala Cys Thr Arg Ser Leu Leu Gly Asp Pro Pro Pro Leu Leu Thr
            795                 800                 805

ctg cca cgg ccc cca cta tca ggg gcc ctg gcc tca atc act gag acc     2562
Leu Pro Arg Pro Pro Leu Ser Gly Ala Leu Ala Ser Ile Thr Glu Thr
        810                 815                 820

atc caa gtc cat cgc aga tac tgg cgc agc tta cgg gtc atg aag gta     2610
Ile Gln Val His Arg Arg Tyr Trp Arg Ser Leu Arg Val Met Lys Val
    825                 830                 835

gaa gac aga gaa gga ccc tcc agt tct aag ttg gtc acc aag aag gca     2658
Glu Asp Arg Glu Gly Pro Ser Ser Ser Lys Leu Val Thr Lys Lys Ala
840                 845                 850                 855

ccc caa cca gcc aaa cct agg tta gct gag cgg atg acc aca cga gaa     2706
Pro Gln Pro Ala Lys Pro Arg Leu Ala Glu Arg Met Thr Thr Arg Glu
                860                 865                 870

aag aag gtt ctg gaa gca ggc atg ggg aaa gtc acc tcg gca tca ttt     2754
Lys Lys Val Leu Glu Ala Gly Met Gly Lys Val Thr Ser Ala Ser Phe
            875                 880                 885

ggg gaa gag tcc act cca ggc cag act aac tca gag aca gct gtg gtg 2802
Gly Glu Glu Ser Thr Pro Gly Gln Thr Asn Ser Glu Thr Ala Val Val
        890                 895                 900
gcc ctc act cag gac cag ccc tca gag gca gcc aca ggg gga gcc act 2850
Ala Leu Thr Gln Asp Gln Pro Ser Glu Ala Ala Thr Gly Gly Ala Thr
    905                 910                 915
ctg gcc cag acc att tct gag gca gcc att ggg gga gcc atg ctg ggc 2898
Leu Ala Gln Thr Ile Ser Glu Ala Ala Ile Gly Gly Ala Met Leu Gly
920                 925                 930                 935
cag acc acc tca gag gag gct gtc ggg gga gcc act ccg gac cag acc 2946
Gln Thr Thr Ser Glu Glu Ala Val Gly Gly Ala Thr Pro Asp Gln Thr
                940                 945                 950
acc tca gag gag act gtg gga gga gcc att ctg gac cag acc acc tca 2994
Thr Ser Glu Glu Thr Val Gly Gly Ala Ile Leu Asp Gln Thr Thr Ser
            955                 960                 965
gag gat gct gtt ggg gga gcc acg ctg ggc cag act acc tca gag gag 3042
Glu Asp Ala Val Gly Gly Ala Thr Leu Gly Gln Thr Thr Ser Glu Glu
        970                 975                 980
gct gta gga gga gct aca ctg gcc cag acc acc tcg gag gca gcc atg 3090
Ala Val Gly Gly Ala Thr Leu Ala Gln Thr Thr Ser Glu Ala Ala Met
    985                 990                 995
gag gga gcc aca ctg gac cag act acg tca gag gag gct cca ggg ggc 3138
Glu Gly Ala Thr Leu Asp Gln Thr Thr Ser Glu Glu Ala Pro Gly Gly
1000                1005                1010                1015
acc gag ctg atc caa act cct cta gcc tcg agc aca gac cac cag acc 3186
Thr Glu Leu Ile Gln Thr Pro Leu Ala Ser Ser Thr Asp His Gln Thr
                1020                1025                1030
ccc cca acc tca cct gtg cag gga act aca ccc cag ata tct ccc agt 3234
Pro Pro Thr Ser Pro Val Gln Gly Thr Thr Pro Gln Ile Ser Pro Ser
            1035                1040                1045
aca ctg att ggg agt ctc agg acc ttg gag cta ggc agc gaa tct cag 3282
Thr Leu Ile Gly Ser Leu Arg Thr Leu Glu Leu Gly Ser Glu Ser Gln
        1050                1055                1060
ggg gcc tca gaa tct cag gcc cca gga gag gag aac cta cta gga gag 3330
Gly Ala Ser Glu Ser Gln Ala Pro Gly Glu Glu Asn Leu Leu Gly Glu
    1065                1070                1075
gca gct gga ggt cag gac atg gct gat tcg atg ctg atg cag gga tct 3378
Ala Ala Gly Gly Gln Asp Met Ala Asp Ser Met Leu Met Gln Gly Ser
1080                1085                1090                1095

agg ggc ctc act gat cag gcc ata ttt tat gct gtg aca cca ctg ccc     3426
Arg Gly Leu Thr Asp Gln Ala Ile Phe Tyr Ala Val Thr Pro Leu Pro
                1100                1105                1110
tgg tgt ccc cat ttg gtg gca gta tgc ccc ata cct gca gca ggc cta     3474
Trp Cys Pro His Leu Val Ala Val Cys Pro Ile Pro Ala Ala Gly Leu
            1115                1120                1125
gac gtg acc caa cct tgt ggg gac tgt gga aca atc caa gag aat tgg     3522
Asp Val Thr Gln Pro Cys Gly Asp Cys Gly Thr Ile Gln Glu Asn Trp
        1130                1135                1140
gtg tgt ctc tct tgc tat cag gtc tac tgt ggt cgt tac atc aat ggc     3570
Val Cys Leu Ser Cys Tyr Gln Val Tyr Cys Gly Arg Tyr Ile Asn Gly
    1145                1150                1155
cac atg ctc caa cac cat gga aat tct gga cac ccg ctg gtc ctc agc     3618
His Met Leu Gln His His Gly Asn Ser Gly His Pro Leu Val Leu Ser
1160                1165                1170                1175
tac atc gac ctg tca gcc tgg tgt tac tac tgt cag gcc tat gtc cac     3666
Tyr Ile Asp Leu Ser Ala Trp Cys Tyr Tyr Cys Gln Ala Tyr Val His
                1180                1185                1190
cac cag gct ctc cta gat gtg aag aac atc gcc cac cag aac aag ttt     3714
His Gln Ala Leu Leu Asp Val Lys Asn Ile Ala His Gln Asn Lys Phe
            1195                1200                1205
ggg gag gat atg ccc cac cca cac taa gccccagaat acggtccctc ttcacct   3768
Gly Glu Asp Met Pro His Pro His Stop
        1210                1215
tctgaggccc acgatagacc agctgtagct cattccagcc tgtaccttgg atgaggggta   3828
gcctcccact gcatcccatc ctgaatatcc tttgcaactc cccaagagtg cttatttaag   3888
tgttaatact tttaagagaa ctgcgacgat taattgtgga tctccccctg cccattgcct    3948
gcttgagggg caccactact ccagcccaga aggaaagggg ggcagctcag tggccccaag   4008
agggagctga tatcatgagg ataacattgg cgggagggga gttaactggc aggcatggca   4068
aggttgcata tgtaataaag tacaagctgt t                                  4099

<210> 2
<211> 1215
<212> PRT
<213> Homo sapiens

<220>

<221>

<222>

<223> Amino acid sequence of histone deacetylase 6

<400> 2

Met Thr Ser Thr Gly Gln Asp Ser Thr Thr Thr Arg Gln Arg Arg Ser
1               5                   10                  15

Arg Gln Asn Pro Gln Ser Pro Pro Gln Asp Ser Ser Val Thr Ser Lys
            20                  25                  30

Arg Asn Ile Lys Lys Gly Ala Val Pro Arg Ser Ile Pro Asn Leu Ala
        35                  40                  45

Glu Val Lys Lys Lys Gly Lys Met Lys Lys Leu Gly Gln Ala Met Glu
    50                  55                  60

Glu Asp Leu Ile Val Gly Leu Gln Gly Met Asp Leu Asn Leu Glu Ala
65                  70                  75                  80

Glu Ala Leu Ala Gly Thr Gly Leu Val Leu Asp Glu Gln Leu Asn Glu
                85                  90                  95

Phe His Cys Leu Trp Asp Asp Ser Phe Pro Glu Gly Pro Glu Arg Leu
            100                 105                 110

His Ala Ile Lys Glu Gln Leu Ile Gln Glu Gly Leu Leu Asp Arg Cys
        115                 120                 125

Val Ser Phe Gln Ala Arg Phe Ala Glu Lys Glu Glu Leu Met Leu Val
    130                 135                 140

His Ser Leu Glu Tyr Ile Asp Leu Met Glu Thr Thr Gln Tyr Met Asn
145                 150                 155                 160

Glu Gly Glu Leu Arg Val Leu Ala Asp Thr Tyr Asp Ser Val Tyr Leu
                165                 170                 175

His Pro Asn Ser Tyr Ser Cys Ala Cys Leu Ala Ser Gly Ser Val Leu
            180                 185                 190

Arg Leu Val Asp Ala Val Leu Gly Ala Glu Ile Arg Asn Gly Met Ala
        195                 200                 205

Ile Ile Arg Pro Pro Gly His His Ala Gln His Ser Leu Met Asp Gly
    210                 215                 220

Tyr Cys Met Phe Asn His Val Ala Val Ala Ala Arg Tyr Ala Gln Gln
225                 230                 235                 240

Lys His Arg Ile Arg Arg Val Leu Ile Val Asp Trp Asp Val His His
                245                 250                 255

Gly Gln Gly Thr Gln Phe Thr Phe Asp Gln Asp Pro Ser Val Leu Tyr
            260                 265                 270
Phe Ser Ile His Arg Tyr Glu Gln Gly Arg Phe Trp Pro His Leu Lys
        275                 280                 285
Ala Ser Asn Trp Ser Thr Thr Gly Phe Gly Gln Gly Gln Gly Tyr Thr
    290                 295                 300
Ile Asn Val Pro Trp Asn Gln Val Gly Met Arg Asp Ala Asp Tyr Ile
305                 310                 315                 320
Ala Ala Phe Leu His Val Leu Leu Pro Val Ala Leu Glu Phe Gln Pro
                325                 330                 335
Gln Leu Val Leu Val Ala Ala Gly Phe Asp Ala Leu Gln Gly Asp Pro
            340                 345                 350
Lys Gly Glu Met Ala Ala Thr Pro Ala Gly Phe Ala Gln Leu Thr His
        355                 360                 365
Leu Leu Met Gly Leu Ala Gly Gly Lys Leu Ile Leu Ser Leu Glu Gly
    370                 375                 380
Gly Tyr Asn Leu Arg Ala Leu Ala Glu Gly Val Ser Ala Ser Leu His
385                 390                 395                 400
Thr Leu Leu Gly Asp Pro Cys Pro Met Leu Glu Ser Pro Gly Ala Pro
                405                 410                 415
Cys Arg Ser Ala Gln Ala Ser Val Ser Cys Ala Leu Glu Ala Leu Glu
            420                 425                 430
Pro Phe Trp Glu Val Leu Val Arg Ser Thr Glu Thr Val Glu Arg Asp
        435                 440                 445
Asn Met Glu Glu Asp Asn Val Glu Glu Ser Glu Glu Glu Gly Pro Trp
    450                 455                 460
Glu Pro Pro Val Leu Pro Ile Leu Thr Trp Pro Val Leu Gln Ser Arg
465                 470                 475                 480
Thr Gly Leu Val Tyr Asp Gln Asn Met Met Asn His Cys Asn Leu Trp
                485                 490                 495
Asp Ser His His Pro Glu Val Pro Gln Arg Ile Leu Arg Ile Met Cys
            500                 505                 510
Arg Leu Glu Glu Leu Gly Leu Ala Gly Arg Cys Leu Thr Leu Thr Pro
        515                 520                 525
Arg Pro Ala Thr Glu Ala Glu Leu Leu Thr Cys His Ser Ala Glu Tyr
    530                 535                 540
Val Gly His Leu Arg Ala Thr Glu Lys Met Lys Thr Arg Glu Leu His
545                 550                 555                 560

Arg Glu Ser Ser Asn Phe Asp Ser Ile Tyr Ile Cys Pro Ser Thr Phe
                565                 570                 575
Ala Cys Ala Gln Leu Ala Thr Gly Ala Ala Cys Arg Leu Val Glu Ala
            580                 585                 590
Val Leu Ser Gly Glu Val Leu Asn Gly Ala Ala Val Val Arg Pro Pro
        595                 600                 605
Gly His His Ala Glu Gln Asp Ala Ala Cys Gly Phe Cys Phe Phe Asn
    610                 615                 620
Ser Val Ala Val Ala Ala Arg His Ala Gln Thr Ile Ser Gly His Ala
625                 630                 635                 640
Leu Arg Ile Leu Ile Val Asp Trp Asp Val His His Gly Asn Gly Thr
                645                 650                 655
Gln His Met Phe Glu Asp Asp Pro Ser Val Leu Tyr Val Ser Leu His
            660                 665                 670
Arg Tyr Asp His Gly Thr Phe Phe Pro Met Gly Asp Glu Gly Ala Ser
        675                 680                 685
Ser Gln Ile Gly Arg Ala Ala Gly Thr Gly Phe Thr Val Asn Val Ala
    690                 695                 700
Trp Asn Gly Pro Arg Met Gly Asp Ala Asp Tyr Leu Ala Ala Trp His
705                 710                 715                 720
Arg Leu Val Leu Pro Ile Ala Tyr Glu Phe Asn Pro Glu Leu Val Leu
                725                 730                 735
Val Ser Ala Gly Phe Asp Ala Ala Arg Gly Asp Pro Leu Gly Gly Cys
            740                 745                 750
Gln Val Ser Pro Glu Gly Tyr Ala His Leu Thr His Leu Leu Met Gly
        755                 760                 765
Leu Ala Ser Gly Arg Ile Ile Leu Ile Leu Glu Gly Gly Tyr Asn Leu
    770                 775                 780
Thr Ser Ile Ser Glu Ser Met Ala Ala Cys Thr Arg Ser Leu Leu Gly
785                 790                 795                 800
Asp Pro Pro Pro Leu Leu Thr Leu Pro Arg Pro Pro Leu Ser Gly Ala
                805                 810                 815
Leu Ala Ser Ile Thr Glu Thr Ile Gln Val His Arg Arg Tyr Trp Arg
            820                 825                 830
Ser Leu Arg Val Met Lys Val Glu Asp Arg Glu Gly Pro Ser Ser Ser
        835                 840                 845
Lys Leu Val Thr Lys Lys Ala Pro Gln Pro Ala Lys Pro Arg Leu Ala
    850                 855                 860

Glu Arg Met Thr Thr Arg Glu Lys Lys Val Leu Glu Ala Gly Met Gly
865                 870                 875                 880
Lys Val Thr Ser Ala Ser Phe Gly Glu Glu Ser Thr Pro Gly Gln Thr
                885                 890                 895
Asn Ser Glu Thr Ala Val Val Ala Leu Thr Gln Asp Gln Pro Ser Glu
            900                 905                 910
Ala Ala Thr Gly Gly Ala Thr Leu Ala Gln Thr Ile Ser Glu Ala Ala
        915                 920                 925
Ile Gly Gly Ala Met Leu Gly Gln Thr Thr Ser Glu Glu Ala Val Gly
    930                 935                 940
Gly Ala Thr Pro Asp Gln Thr Thr Ser Glu Glu Thr Val Gly Gly Ala
945                 950                 955                 960
Ile Leu Asp Gln Thr Thr Ser Glu Asp Ala Val Gly Gly Ala Thr Leu
                965                 970                 975
Gly Gln Thr Thr Ser Glu Glu Ala Val Gly Gly Ala Thr Leu Ala Gln
            980                 985                 990
Thr Thr Ser Glu Ala Ala Met Glu Gly Ala Thr Leu Asp Gln Thr Thr
        995                 1000                1005
Ser Glu Glu Ala Pro Gly Gly Thr Glu Leu Ile Gln Thr Pro Leu Ala
    1010                1015                1020
Ser Ser Thr Asp His Gln Thr Pro Pro Thr Ser Pro Val Gln Gly Thr
1025                1030                1035                1040
Thr Pro Gln Ile Ser Pro Ser Thr Leu Ile Gly Ser Leu Arg Thr Leu
                1045                1050                1055
Glu Leu Gly Ser Glu Ser Gln Gly Ala Ser Glu Ser Gln Ala Pro Gly
            1060                1065                1070
Glu Glu Asn Leu Leu Gly Glu Ala Ala Gly Gly Gln Asp Met Ala Asp
        1075                1080                1085
Ser Met Leu Met Gln Gly Ser Arg Gly Leu Thr Asp Gln Ala Ile Phe
    1090                1095                1100
Tyr Ala Val Thr Pro Leu Pro Trp Cys Pro His Leu Val Ala Val Cys
1105                1110                1115                1120
Pro Ile Pro Ala Ala Gly Leu Asp Val Thr Gln Pro Cys Gly Asp Cys
                1125                1130                1135
Gly Thr Ile Gln Glu Asn Trp Val Cys Leu Ser Cys Tyr Gln Val Tyr
            1140                1145                1150
Cys Gly Arg Tyr Ile Asn Gly His Met Leu Gln His His Gly Asn Ser
        1155                1160                1165

Gly His Pro Leu Val Leu Ser Tyr Ile Asp Leu Ser Ala Trp Cys Tyr
    1170                    1175                    1180
Tyr Cys Gln Ala Tyr Val His His Gln Ala Leu Leu Asp Val Lys Asn
1185                    1190                    1195                    1200
Ile Ala His Gln Asn Lys Phe Gly Glu Asp Met Pro His Pro His
                    1205                    1210                    1215

<210> 3
<211> 20
<212> DNA
<213> Artificial sequence

<220>
<221>
<222>
<223> Antisense oligonucleotide no.1

<400> 3
catagttgag gaggcttggc                                                    20

<210> 4
<211> 20
<212> DNA
<213> Artificial sequence

<220>
<221>
<222>
<223> Antisense oligonucleotide no.2

<400> 4
tcctggccgg ttgaggtcat                                                    20

<210> 5

<211> 20
<212> DNA
<213> Artificial sequence

<220>
<221>
<222>
<223> Antisense oligonucleotide no.3

<400> 5
ggttgaggtc atagttgagg                                                    20

<210> 6
<211> 20
<212> DNA
<213> Artificial sequence

<220>
<221>
<222>
<223> Antisense oligonucleotide no.4

<400> 6
ccctgccgcg gttcttccac                                                    20

<210> 7
<211> 20
<212> DNA
<213> Artificial sequence

<220>
<221>
<222>

<223> Antisense oligonucleotide no.5

<400> 7
gctgcctggt tgtggtggaa                                                    20

<210> 8
<211> 20
<212> DNA
<213> Artificial sequence

<220>
<221>
<222>
<223> Antisense oligonucleotide no.6

<400> 8
tttattacat atgcaacctt                                                    20

<210> 9
<211> 20
<212> DNA
<213> Artificial sequence

<220>
<221>
<222>
<223> Antisense oligonucleotide no.7

<400> 9
aacagcttgt actttattac                                                    20

<210> 10
<211> 20

<212> DNA
<213> Artificial sequence

<220>
<221>
<222>
<223> Antisense oligonucleotide no.8

<400> 10
ccttgaccgt ggtgcacatc                                                    20

<210> 11
<211> 20
<212> DNA
<213> Artificial sequence

<220>
<221>
<222>
<223> Antisense oligonucleotide no.9

<400> 11
gtggtgcaca tcccaatcta                                                    20

<210> 12
<211> 20
<212> DNA
<213> Artificial sequence

<220>
<221>
<222>
<223> Antisense oligonucleotide no.10

<400> 12
ccattaccgt ggtggacatc                                                    20

<210> 13
<211> 20
<212> DNA
<213> Artificial sequence

<220>
<221>
<222>
<223> Antisense oligonucleotide no.11

<400> 13
gtggtggaca tcccaatcca                                                    20

<210> 14
<211> 20
<212> DNA
<213> Artificial sequence

<220>
<221>
<222>
<223> Antisense oligonucleotide no.12

<400> 14
cgctcctcag ggactgcccc                                                    20

<210> 15
<211> 20
<212> DNA

<213> Artificial sequence

<220>
<221>
<222>
<223> Antisense oligonucleotide no.13

<400> 15
acatcagctc ttccttttca                                                    20

<210> 16
<211> 20
<212> DNA
<213> Artificial sequence

<220>
<221>
<222>
<223> Antisense oligonucleotide no.14

<400> 16
ccaaggcaca ttgatggtat                                                    20

<210> 17
<211> 20
<212> DNA
<213> Artificial sequence

<220>
<221>
<222>
<223> Antisense oligonucleotide no.15

<400> 17
actggccatg tcaggattgg                                                    20

<210> 18
<211> 20
<212> DNA
<213> Artificial sequence

<220>
<221>
<222>
<223> Antisense oligonucleotide no.16

<400> 18
tccgtagggc atgcccactg                                                    20

<210> 19
<211> 20
<212> DNA
<213> Artificial sequence

<220>
<221>
<222>
<223> Antisense oligonucleotide no.17

<400> 19
tggcagggtc agcaggggtg                                                    20

<210> 20
<211> 20
<212> DNA
<213> Artificial sequence

<220>
<221>
<222>
<223> Antisense oligonucleotide no.18

<400> 20
agcgtggctc ccccaacagc                                                    20

<210> 21
<211> 20
<212> DNA
<213> Artificial sequence

<220>
<221>
<222>
<223> Antisense oligonucleotide no.19

<400> 21
aattctcttg gattgttcca                                                    20

<210> 22
<211> 20
<212> DNA
<213> Artificial sequence

<220>
<221>
<222>
<223> Antisense oligonucleotide no.20

<400> 22

tatcagctcc ctcttggggc                                                20

<210> 23

<211> 21

<212> DNA

<213> Artificial sequence

<220>

<221>

<222>

<223> Head reverse primer

<400> 23
cctcaactat gacctcaacc g                                               21

<210> 24

<211> 20

<212> DNA

<213> Artificial sequence

<220>

<221>

<222>

<223> Head forward primer

<400> 24
tgtcctcctc catgttgtcc                                                20

<210> 25

<211> 20

<212> DNA

<213> Artificial sequence

<220>
<221>
<222>
<223> Mid reverse primer

<400> 25
gctgatcctg tctctggagg                                                    20

<210> 26
<211> 21
<212> DNA
<213> Artificial sequence

<220>
<221>
<222>
<223> Mid forward primer

<400> 26
tggtgaccaa cttagaactg g                                                  21

<210> 27
<211> 20
<212> DNA
<213> Artificial sequence

<220>
<221>
<222>
<223> Tail reverse primer

<400> 27
aagttggtca ccaagaaggc                                                    20

<210> 28
<211> 20
<212> DNA
<213> Artificial sequence

<220>
<221>
<222>
<223> Tail forward primer

<400> 28
aggctggaat gagctacagc                                                    20

<210> 29
<211> 36
<212> DNA
<213> Artificial sequence

<220>
<221>
<222>
<223> His6 linker

<400> 29
ctagatgccg cggggttctc atcatcatca tcatca                                  36

<210> 30
<211> 34
<212> DNA
<213> Artificial sequence

<220>

<221>

<222>

<223> His6 linker

<400> 30
tatgatgatg atgatgatga gaaccccgcg gcat                                34

<210> 31
<211> 20
<212> DNA
<213> Artificial sequence

<220>
<221>
<222>
<223> Control oligonucleotide

<400> 31
cctcttacct cagttacaat                                                20

## Description

### BACKGROUND OF INVENTION

### 1. Field of Invention

The present invention relates to an apoptosis inducer and a method of screening it. The apoptosis inducer is promising as an anti-cancer agent.

### 2. Related Art

It has been found that histone deacetylase (HDAC) plays an important role in the regulation of gene expression (Khochbin S., Verdel A., Lemercier C., Seigneurin-Berny D., Functional significance of histone deacetylase diversity, Curr. Opin. Genet. Dev. 2001 Apr; 11(2) 162-6). For histone deacetylases in human, there are many isozymes, which are classified into class I (HDAC isozymes 1, 2, 3, and 8), class II (HDAC isozymes 4, 5, 6, and 7), and class III (SIRT isozymes 1, 2, 3, 4, 5, 6, and 7). Among them, histone deacetylase 6 (HDAC6) is existed in the cytoplasm unlike the other isozymes that are existed in the nucleus. Thus it has a property different from the other isozymes in that histone cannot be its natural substrate, it is inhibited by trichostatin A, it has a unique structure, and the like.

It is known that microtubules that play important roles in cell division contain α-tubulin as a constitutive protein thereof, and the protein undergoes acetylation when microtubules are stabilized with taxol etc. (Piperno, G., LeDizet, M. & Chang, X. -j. (1987) Microtubules containing acetylated α-tubulin in mammalian cells in culture, J. Cell Biol. 104, 289-302). Thus, it is thought that, in the cytoplasm, there are enzymes (α-tubulin-acetylating enzymes) (α-tubulin acetylase) that add an acetyl group to α-tubulin, and enzymes (α-tubulin-deacetylating enzymes) (α-tubulin deacetylase) that remove an acetyl group added to α-tubulin.

It is known that the acetylation of α-tubulin contributes to stabilizing microtubules (Piperno, G., LeDizet, M. & Chang, X. -j. (1987) Microtubules containing acetylated α-tubulin in mammalian cells in culture, J. Cell Biol. 104, 289-302). However, although histone deacetylase 6 (HDAC6) is a deacetylating enzyme for α-tubulin, it is not known that apoptosis is induced in the cell by inhibiting HDAC6.

### SUMMARY OF INVENTION

The present invention intends to provide an apoptosis inducer that generates an anti-cancer effect by inducing apoptosis, and an anti-cancer agent comprising said apoptosis inducer as an active ingredient, as well as a method of screening an apoptosis inducer.

The present inventors have focused on the facts that among the variety of isozymes of histone deacetylases (HDAC), only histone deacetylase 6 (HDAC6) exist in the cytoplasm and its natural substrate is not histone, and, after intensive and extensive research, have confirmed that HDAC6 is a deacetylating enzyme of α-tubulin. The present inventors have further found that apoptosis can be induced in the cell by inhibiting HDAC6 to enhance the acetylation of α-tubulin thereby stabilizing microtubules, and therefore, have completed the present invention.

Thus the present invention provides an apoptosis inducer comprising a substance that inhibits histone deacetylase 6 (HDAC6). The substance that inhibits histone deacetylase 6 (HDAC6) may be a substance that inhibits histone deacetylase 6 (HDAC6) per se or that suppresses the production (synthesis) of histone deacetylase 6 (HDAC6). As an example of a substance that suppresses the synthesis of histone deacetylase 6, there may be mentioned a nucleic acid or a peptide, and as the nucleic acid there may be mentioned an antisense oligonucleotide or a ribozyme of histone deacetylase 6 (HDAC6).

The present invention also provides a method of screening an apoptosis inducer that inhibits HDAC6, and more specifically a method of screening an apoptosis inducer comprising the steps of:
(1) determining whether or not a test substance inhibits histone deacetylase 6 (HDAC6) by using deacetylation of an acetylated substance that can be a substrate for histone deacetylase 6 (HDAC6) or decrease in the expression of histone deacetylase 6 (HDAC6) as an index; and
(2) further confirming, when inhibition occurs, whether it induces apoptosis of the cell in vitro and/or in vivo.

### BRIEF EXPLANATION OF THE DRAWINGS

Figure 1 is a graph showing that recombinantly produced HDAC6 according to the present invention has a deacetylating activity, and the activity is inhibited by Trichostatin A.

Figure 2 is a drawing of Western blot that shows that HDAC6 deacetylates acetylated α-tubulin.

Figure 3 is a drawing of Western blot that shows that some of the tested HDAC6 antisense oligonucleotides inhibit the expression of HDAC6 and enhance the acetylation of α-tubulin.

Figure 4 is a drawing of Western blot that shows that HDAC6 antisense oligonucleotide Nos. 4, 5, 6 or 16 inhibits the production of HDAC6 and enhances the acetylation of α-tubulin by changing treatment time.

Figure 5 is a microgram that shows that the introduction of HDAC6 antisense oligonucleotide No. 4 of the present invention induces the microtubule polymerization in cytoplasm on HeLa cells.

Figure 6 is a graph showing that HDAC6 antisense oligonucleotides No. 4, 5, or 16 of the present invention induces apoptosis in HeLa cells when judged by Annexin V positivity.

Figure 7 is a graph showing that HDAC6 antisense oligonucleotides No. 4, 5, or 16 of the present invention induces mitochondrial depolarization in HeLa cells when judged by JC-1 staining.

Figure 8 is a drawing of Western blot that shows that the HDAC6 antisense oligonucleotide No. 16 of the present invention inhibits the production of HDAC6 and enhances the acetylation of α-tubulin in various human cancer cells.

Figure 9 is a graph showing that the HDAC6 antisense oligonucleotides No. 16 of the present invention induces mitochondrial depolarization in various human cancer cells when judged by JC-1 staining.

Figure 10 is a graph showing that the HDAC6 antisense oligonucleotide No. 16 of the present invention induces the cell death in various human cancer cells.

Figure 11 is a micrograph that shows that the HDAC6 antisense oligonucleotide No. 16 of the present invention stabilizes the microtubules and inhibits disappearance of the microtubules by Colcemid.

Figure 12 shows the cloning of the full-length HDAC6 cDNA and the construction of a transfer vector for baculovirus.

Figure 13 is continued from Figure 12.

Figure 14 shows the construction of a vector for expression of HDAC6 in animal cells.

### EMBODIMENTS FOR CARRYING OUT THE INVENTION

In accordance with the present invention, it was firstly confirmed that HDAC6 is a deacetylating enzyme of α-tubulin. For this purpose, a cDNA encoding HDAC6 was cloned into mammalian expression vector (Example 1), this vector was introduced into a host cell to overexpress HDAC6 in the cell, and the degree of acetylation of α-tubulin was observed. If HDAC6 is a deacetylating enzyme of α-tubulin, the acetyl group of α-tubulin should be removed by HDAC6 overexpress and thereby the amount of acetylated α-tubulin in the cell should be decreased. The nucleotide sequence of cDNA encoding HDAC6 is set forth in SEQ ID NO: 1 and the amino acid sequence encoded thereby is set forth in SEQ ID NO: 2.

As a result, when HDAC6 was overexpress, the degree of acetylation was decreased by 50%, whereas when the vector that does not express HDAC6 (His6-HDAC6(full)/pcDNA3.1(+)AS) or a vector alone was introduced into the cell, the degree of acetylation of α-tubulin was not decreased. As a result, it was demonstrated that HDAC6 is a deacetylating enzyme of α-tubulin.

Then, methods of decreasing the expression of HDAC6 in the cell were investigated. As a method of inhibiting the expression of the HDAC6 gene, by way of example, the inhibition of expression was attempted using a phosphorothioated oligonucleotide complementary to mRNA (antisense oligonucleotide) of HDAC6. As the antisense oligonucleotide, three oligonucleotides that are complementary to sequences comprising the translation initiation codon ATG of HDAC6, i.e. antisense oligonucleotide 1: CATAGTTGAG GAGGCTTGGC (SEQ ID No. 3), antisense oligonucleotide 2: TCCTGGCCGG TTGAGGTCAT (SEQ ID No. 4), and antisense oligonucleotide 3: GGTTGAGGTC ATAGTTGAGG (SEQ ID No. 5); as well as antisense oligonucleotide 4: CCCTGCCGCG GTTCTTCCAC (SEQ ID No. 6) complementary to a sequence upstream of ATG, and antisense oligonucleotide 5: GCTGCCTGGT TGTGGTGGAA (SEQ ID No. 7) complementary to a sequence downstream of ATG were used.

Furthermore, antisense oligonucleotide 6: TTTATTACAT ATGCAACCTT (SEQ ID NO: 8) complementary to an upstream sequence comprising the polyadenylation signal sequence (bases 4082-4087 in SEQ ID NO: 1), and antisense oligonucleotide 7: AACAGCTTGT ACTTTATTAC (SEQ ID NO: 9) complementary to a downstream sequence comprising the polyadenylation signal sequence, antisense oligonucleotide 8: CCTTGACCGT GGTGCACATC (SEQ ID NO: 10) complementary to a downstream sequence comprising a sequence corresponding to the conserved aspartic acid and histidine residues (DH) in the first catalytic domain (nucleotides 352-1305 in SEQ ID NO: 1) and antisense oligonucleotide 9: GTGGTGCACA TCCCAATCTA (SEQ ID NO: 11) complementary to an upstream sequence comprising a sequence corresponding to the above DH, as well as antisense oligonucleotide 10: CCATTACCGT GGTGGACATC (SEQ ID NO: 12) complementary to a downstream sequence comprising a sequence corresponding to the conserved DH in the second catalytic domain (nucleotides 1537-2475 in SEQ ID NO: 1) and antisense oligonucleotide 11: GTGGTGGACA TCCCAATCCA (SEQ ID NO: 13) complementary to an upstream sequence comprising a sequence corresponding to the above DH were used.

Furthermore, in order to cover the entire region of HDAC6 mRNA, antisense oligonucleotide 12: CGCTCCTCAG GGGACTGCCC (SEQ ID NO: 14) complementary to a sequence comprising nucleotides 1-20 of SEQ ID NO: 1, antisense oligonucleotide 13: ACATCAGCTC TTCCTTTTCA (SEQ ID NO: 15) complementary to a sequence comprising nucleotides 501-520, antisense oligonucleotide 14: CCAAGGCACA TTGATGGTAT (SEQ ID NO: 16) complementary to a sequence comprising nucleotides 1001-1020, and antisense oligonucleotide 15: ACTGGCCATG TCAGGATTGG (SEQ ID NO: 17) complementary to a sequence comprising nucleotides 1501-1520, antisense oligonucleotide 16: TCCGTAGGGC ATGCCCACTG (SEQ ID NO: 18) complementary to a sequence comprising nucleotides 2001-2020, antisense oligonucleotide 17: TGGCAGGGTC AGCAGGGGTG (SEQ ID NO: 19) complementary to a sequence comprising nucleotides 2501-2520, antisense oligonucleotide 18: AGCGTGGCTC CCCCAACAGC (SEQ ID NO: 20) complementary to a sequence comprising nucleotides 3001-3020, antisense oligonucleotide 19: AATTCTCTTG GATTGTTCCA (SEQ ID NO: 21) complementary to a sequence comprising nucleotides 3501-3520, and antisense oligonucleotide 20: TATCAGCTCC CTCTTGGGGC (SEQ ID NO: 22) complementary to a sequence comprising nucleotides 4001-4020 were used.

As a result, the effect of suppressing the gene expression, i.e. reducing the production of HDAC6 was exhibited by using antisense oligonucleotides 1 to 5, antisense oligonucleotides 8 to 11, and antisense oligonucleotide 16. Also, enhanced acetylation of α-tubulin was confirmed concomitantly with the decreased production of HDAC6.

Thus, when an antisense oligonucleotide for which the effect of enhancing the acetylation of α-tubulin was confirmed was introduced into cancer cells, it was observed, microtubules stabilized in the cytoplasm and apoptosis were present. Trichostatin A that is known as a HDAC inhibitor was also confirmed to provide the similar effect.

From the above experimental results, it can be reasonably estimated that a substance that inhibits HDAC6 induces apoptosis and is useful as an anti-cancer agent. Thus, by selecting a substance that inhibits the activity of HDAC6 using the deacetylation and/or acetylation of α-tubulin as an index, it is possible to search an apoptosis inducer and an anti-cancer agent.

Since, as substances that inhibit HDAC6, there may be contemplated enzyme inhibitors that inhibit the enzyme HDAC6 and substances that suppress the production (synthesis) of HDAC6, there are methods of searching an enzyme inhibitor that inhibits the enzyme HDAC6 and methods of searching a substance that inhibits the production (synthesis) of HDAC6.

In order to search an enzyme inhibitor of HDAC6, the enzyme HDAC6, an acetylated substance that can be substrate for HDAC 6, and a test substance are reacted, and the deacetylation of the substrate is observed as an index. In the case where the test substance inhibits the deacetylation, the test substance is expected as an apoptosis inducer. Acetylated substances which can be substrates for HDAC6 are, for example, acetylated α-tublin, an acetylated histone, or synthetic peptides in which a lysine residue has been acetylated. As a method for detecting a deacetylation, any method which can be used for detection of deacetylation may be used, and for example, in the case where the acetylated α-tublin is used as the substrate, a decrease of the acetylated α-tublin and/or an increase of released acetate or deacetylated α-tublin are measured. In addition, for screening of enzyme inhibitors, HDAC Fluorescent activity Assay Kit, AK-500 (BIOMOL), HDAC Assay Kit (CycLex) may be used.

Furthermore, in order to search a substance that inhibits the expression of HDAC6, cells such as an animal cell expressesing HDAC6 is cultured in the presence or in the absense of the test substance, and when the amount of protein and/or mRNA of HDAC6 in the cell cultured in the presence of the test substance is decreased compared to that of protein and/or mRNA of HDAC6 in the cell cultured in the absence of the test substance, the test substance is an expression-suppressant, and may be judged to be an apoptosis inducer.

The amount of protein of HDAC6 can be determined by the Western blot method etc., and the amount of mRNA can be determined by the Northern blot method or the RT-PCR method, etc. When the cell is cultured in the presence or in the absence of the test substance, and the ratio of acetylated α-tubulin to α-tubulin in the cell cultured in the presence of the test substance is compared. When the ratio of acetylated α-tubulin in the cell cultured in the presence of the test substance is higher than the ratio of acetylated α-tubulin in the cell cultured in the absence of the test substance, the test substance is an expression suppressant of HDAC6, and may be estimated to be an apoptosis inducer.

As the above cells, any cells that express HDAC6 can be used, and include animal cells, preferably cultured human cells, and most preferably cancer cells, and there may be mentioned HeLa cells, HL-60 cells, A549 cells, HepG2 cells, LoVo cells, SW480 cells, Calu-1 cells, and more preferably HeLa cell, HepG2 cell, LoVo cell, SW480 cell, Calu-1 cell. Also, genetically engineered cells may be used in which DNA encoding cloned HDAC6 has been artificially introduced so as to express HDAC6 in excess.

### EXAMPLES

The present invention will now be explained in more detail with reference to the Examples.

### Example 1.

### Isolation of total RNA

A cDNA encoding HDAC6 was cloned by dividing the coding region of HDAC6 into three parts. HL-60 cells (ATCC) were cultured in RPMI 1640 medium containing 10% FCS and were centrifuged at 500 x g for 5 minutes to collect the cells. The collected 1 x 10⁶ cells were lysed with 1 ml of TRIzole reagent (Gibco BRL), to which 200 µl of chloroform was added and suspended, and the aqueous layer was collected. To the collected aqueous layer was added 500 µl of isopropyl alcohol and mixed, which was centrifuged at 12000 x g for 10 minutes to precipitate total RNA. The precipitated total RNA was rinsed with 75% ethanol, and was slightly air-dried and then dissolved in 50 µl of water. The concentration of RNA was measured at a wavelength of 260 nm and was stored at -80°C until use.

### Cloning of cDNA encoding HDAC6

The isolated total RNA of HL-60 cells was used for a reverse transcription reaction. For the reaction, the SuperScript II of Gibco BRL was used, and 0.5 µg of Random 9 mers included in TaKaRa RNA PCR Kit (AMV) Ver. 2.1 was used as the primer, and treated as described in the instruction attached to Superscript II to obtain total cDNA which was used as a template for PCR.
For cloning of the upstream (HEAD) region 
5'-side (Reverse) primer: CCTCAACTAT GACCTCAACC G
(SEQ ID NO: 23)
3'-side (Forward) primer: TGTCCTCCTC CATGTTGTCC (SEQ
ID NO: 24)
 For cloning of the middle stream (MID) region 
5'-side (Reverse) primer: GCTGATCCTG TCTCTGGAGG (SEQ
ID NO: 25)
3'-side (Forward) primer: TGGTGACCAA CTTAGAACTG G
(SEQ ID NO: 26)
 For cloning of the downstream (TAIL) region

5'-side (Reverse) primer: AAGTTGGTCA CCAAGAAGGC (SEQ
ID NO: 27)
3'-side (Forward) primer: AGGCTGGAAT GAGCTACAGC (SEQ
ID NO: 28)

With the above total cDNA as template, PCR was performed using the above primer pairs and using LA Taq (Takara) as the polymerase for 30 cycles with each cycle comprising 94°C for 1 minute, 55°C for 30 seconds, and 72°C for 2 minutes. Each cDNA thus amplified was subcloned into a cloning vector pT7blue T-Vector (Novagen) to obtain HDAC6 HEAD/pT7blue, HDAC6 MID/pT7blue, and HDAC6 TAIL/pT7blue, which were then sequenced.

His6 linkers: CTAGATGCCG CGGGGTTCTC ATCATCATCA TCATCA (SEQ ID NO: 29) and TATGATGATG ATGATGATGA GAACCCCGCG GCAT (SEQ ID NO: 30) were phosphorylated with T4 polynucleotide kinase, annealed and then inserted into the XbaI/NdeI sites of the above HDAC6 HEAD/pT7blue, and the vector constructed was digested with XbaI and DraIII to obtain a His6-tagged HEAD(Xba/DraIII) fragment. This His6-tagged HEAD(Xba/DraIII) fragment and a MID(DraIII/Bpu1102I) fragment obtained by digesting the above HDAC6 MID/pT7blue with DraIII and Bpu1102I were inserted into the above HDAC6 TAIL/pT7blue that had been digested with XbaI and DraIII to construct a full-length HDAC6 (His6 fused at N-terminal)/pT7blue (designated as His6-HDAC6(full)/pT7blue).

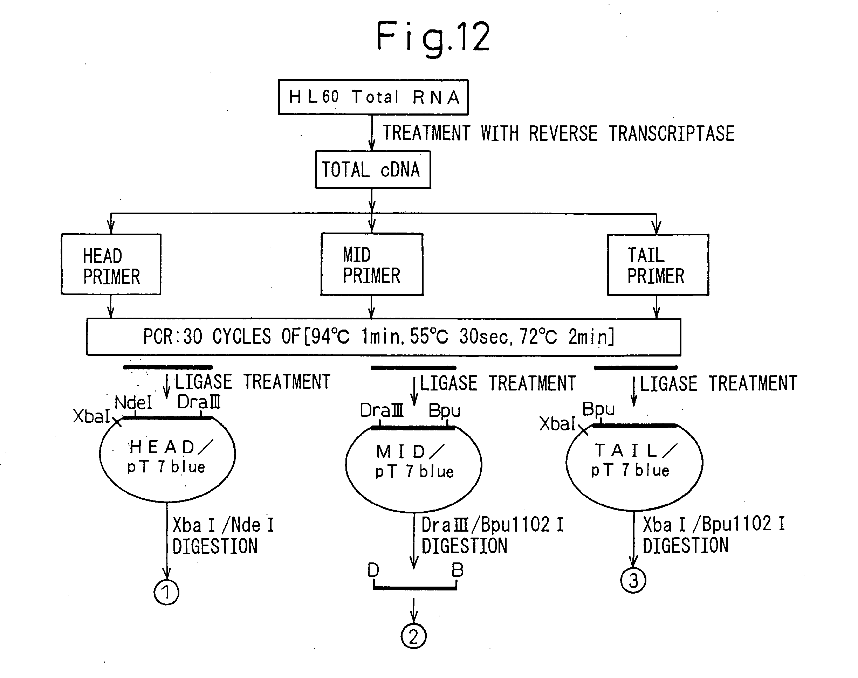

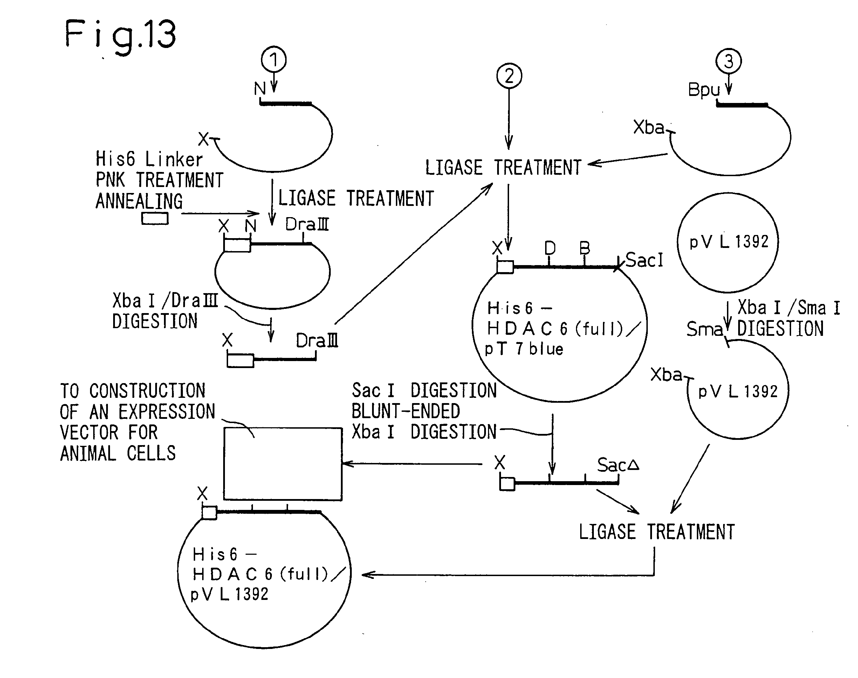

The above His6-HDAC6(full)/pT7blue was digested with SacI, blunt-ended with T4 DNA polymerase, and then digested with XbaI. The fragment obtained was ligated to the XbaI/SmaI site of the vector pVL1392 to construct a transfer vector His6-HDAC6(full)/pVL1392. The above process is described in Figures 12 and 13.

Then according to a standard method, this transfer vector was transfected into the Sf-9 cells cultured in the TNM-FH medium. Thus, 4 µg of the above transfer vector His6-HDAC6(full)/pVL1392, 0.5 µg of the Bac-N-Blue linearized AcMNPV DNA (Invitrogen) and 20 µl of the InsectinPlus liposome were incubated with 2 x 10⁶ of Sf-9 cells in one ml of the Grace's insect medium according to the instruction attached to the kit. After cultivating for 7 days, the culture supernatant was collected, subjected to plaque purification, and then the virus was amplified from a single plaque to prepare a high-titer virus stock.

### Example 2. Production of recombinant HDAC6 protein

The virus (about 10⁸ pfu/ml) amplified in Example 1 was diluted 1/100 and was infected to 300 ml of Sf-9 cells (10⁶ cells/ml) at a MOI = 1, which was then cultured under stirring at 70 rpm at 27°C for 72 hours. After culturing, the cells were collected by centrifuging at 500 x g for 5 minutes and stored at -80°C. The cells were suspended into 6 ml of buffer A (50 mM sodium phosphate buffer, pH 7.5, 300 mM NaCl, 20 mM imidazole) containing protease inhibitors (1 mM PMSF, 2 µM leupeptin, 2 µM pepstatin, 200 nM aprotinin), homogenized at 20 strokes by a Teflon homogenizer, and the homogenate was centrifuged at 20000 X g at 4°C for 30 minutes to obtain a clear cell homogenate. To the cell homogenate obtained, one ml of an adsorbing resin Ni-NTA Superflow (Qiagen) that had been equilibrated with buffer A was added to allow for adsorption at 4°C for 1 to 2 hours.

The above solution containing the absorbing resin was centrifuged at 2000 X g at 4°C for 5 minutes to remove the supernatant, and the recovered the adsorbing resin was resuspended in 10 ml of buffer A, which is centrifuged at 2000 X g at 4°C for 5 minutes and the supernatant was discarded. The procedure was repeated for three times. The washed resin was suspended in buffer A, and then filled into a column (Econo column, BIO-RAD) and washed with 20 ml of buffer A. The column was eluted with buffer B (50 mM sodium phosphate, pH 7.5, 300 mM NaCl, 300 mM imidazole), and the eluted solution was collected at fractions of 1 ml, which were dialyzed against the dialysis buffer (50 mM Tris-Cl, pH 7.5, 150 mM NaCl, 2 mM EDTA). Protein in the dialysate was determined using Protein Assay kit (BIO-RAD) and stored at -80°C.

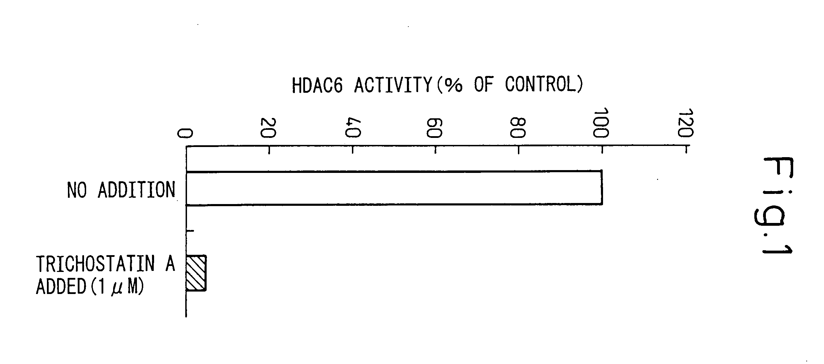

The deacetylating activity of the HDAC6 protein purified as above was determined using HDAC Fluorescent Activity Assay/Drug Discovery Kit (BIOMOL). The determination was also carried out when 1 µM of Trichostatin A, an inhibitor of HDAC6, was presented to the assay system and the result shown in Figure 1 was obtained. Thus, The deacetylating activity of the test sample was completely inhibited by Trichostatin A, confirming that the recombinantly prepared HDAC6 has the inherent deacetylating activity.

### Example 3. Construction of an expression vector for forced expression of HDAC6 and the promotion of deacetylation of α-tubulin by the forced expression of HDAC6

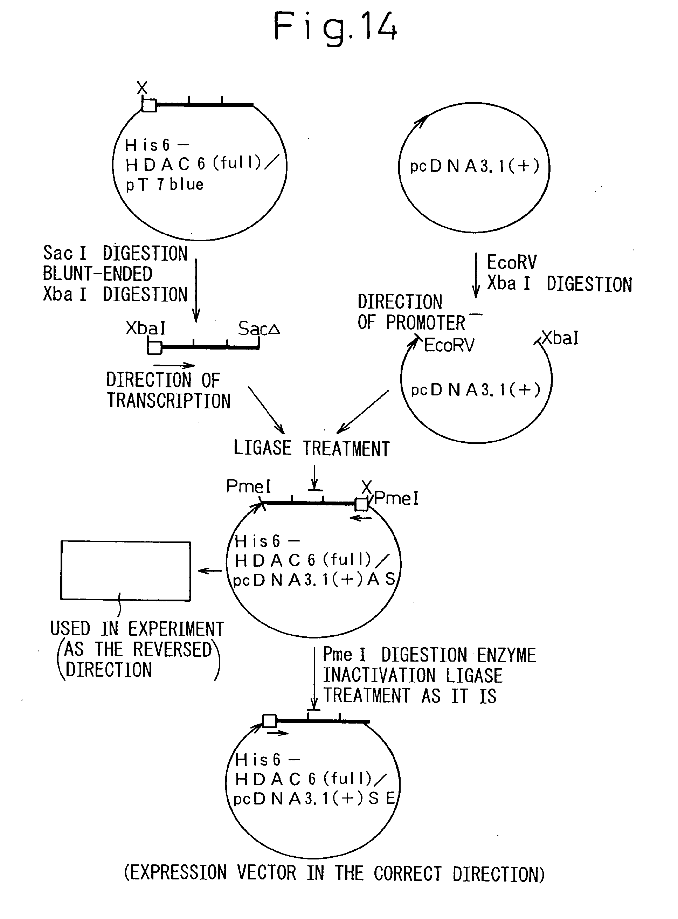

The His6-HDAC6(full)/pT7blue constructed in Example 1 was digested with SacI, blunt-ended with T4 DNA polymerase, and digested with XbaI, and this fragment was inserted into pcDNA3.1(+) (Invitrogen) that had been digested with EcoRV and XbaI to obtain His6-HDAC6(full)/pcDNA3.1(+)AS. Since HDAC6 cDNA was inserted in the reverse direction to the promoter for expression in the constructed vector, said vector was digested with PmeI, and PmeI was inactivated, under which condition pcDNA3.1(+) and HDAC6 fragment were ligated to obtain an expression vector, His6-HDAC6(full)/pcDNA3.1(+)SE, for the forced expression of HDAC6, in which HDAC6 cDNA was inserted in the correct direction to the promoter for expression. The above process is described in Figure 14.

In a 10-cm tissue culture dish, HeLa cells were plated with the MEM medium containing 10% FCS and 1 X nonessential amino acids, 5 µg of His6-HDAC6(full)/pcDNA3.1(+)SE that had been constructed so as to permit the expression of HDAC6 as a histidine-fused protein and 5 µg of pEGFP-Cl (Clontech) were co-transferred using the FuGENE6 transfection reagent (Roche). A similar procedure was followed using His6-HDAC6(full)/pcDNA3.1(+)AS and pcDNA3.1(+). After 48 hours, cells were detached with trypsin and recovered. Using the FACS caliber (Becton Dickinson), only EGFP (Enhanced Green Fluorecence Protein)-positive cells were separately collected.

The recovered cells were lysed with the SDS-PAGE sample buffer (bromophenol blue-free), and protein concentration was determined. After the determination, the cell lysate at a protein amount of 2.5 µg/lane was separated by SDS-PAGE, it was transferred to a PVDF membrane, and was immnoblotted with anti-acetylated α-tubulin antibody (clone 6-11B-1, Sigma), anti-α-tubulin antibody (clone DM1A, Sigma) or anti-RGS His antibody (QIAGEN).

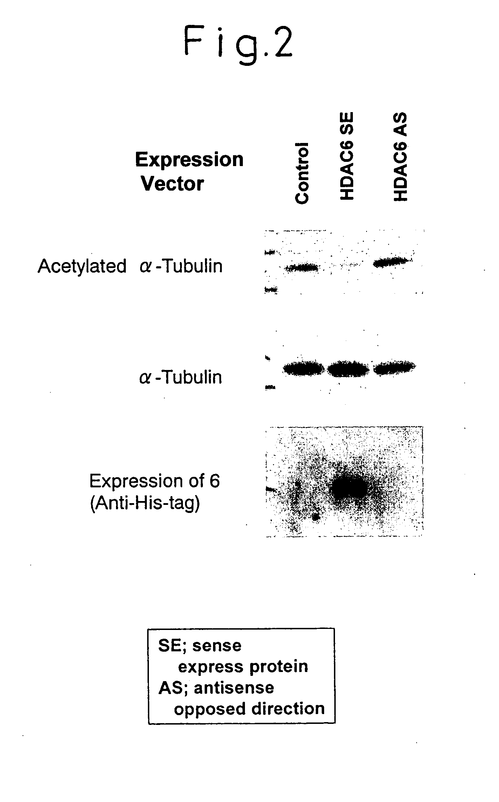

As shown in Figure 2, there were no changes in the degree of acetylation of α-tubulin in the cells into which His6-HDAC6(full)/pcDNA3.1(+)AS or pcDNA3.1(+), a vector that does not express HDAC6, was introduced, whereas in the cells in which HDAC6 was forcedly expressed the degree of acetylation of α-tubulin was decreased, suggesting that HDAC6 plays a role in the deacetylation of α-tubulin.

### Example 4. Suppression of HDAC6 protein expression using phosphorothioated antisense oligonucleotide (antisense oligonucleotide) and the result enhancement of acetylation of α-tubulin

Based on the mRNA sequence of the HDAC6 gene (Gene Bank Accession No. NM_006044), 20 different antisense oligonucleotides (SEQ ID NO: 3 to 22) and control oligonucleotide: CCTCTTACCT CAGTTACAAT (SEQ ID NO: 31) (this is derived from a sequence resulting from abnormal splicing at site 705 in pre-mRNA of erythrocyte β globin in hemoglobinopathy thalassanemia, and this oligonucleotide does not have specific targeting sites or biological activity in normal cells) were designed, and were synthesized as phosphorothioated antisense oligonucleotides (Sawady Technology).

The each synthesized antisense oligonucleotide was dissolved in sterile water, and was introduced into HeLa cells using the OligofectAMINE (Invitrogen) according to an attached protocol. The 1.5 X 10⁵ HeLa cells were plated in a 6-well plate, the final concentration of antisense oligonucleotide was set at 200 nM, and OligofectANINE was used at 3 µl/well.

Forty eight hours after the introduction, the cells were detached with trypsin and collected. After the cells were lysed with a SDS-PAGE sample buffer (BPB-free) and protein concentration was determined, the cell lysate at 25 µg/lane was separated by SDS-PAGE, transferred to a PVDF membrane, and immunoblotted with anti-acetylated α-tubulin antibody (clone 6-11B-1, Sigma), anti-α-tubulin antibody (clone DM1A, Sigma) or anti-HDAC6 antibody (H-300, Santa Cruz).

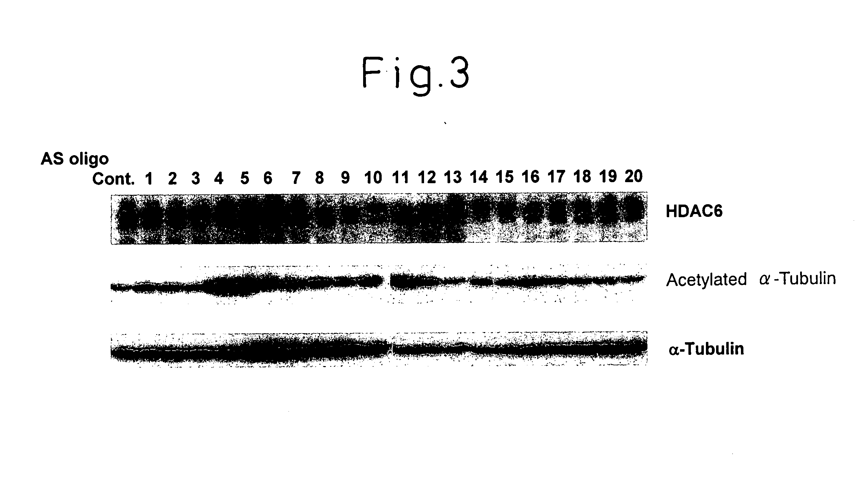

As shown in Figure 3, about half of the 20 antisense oligonucleotides tested caused the reduction in the amount of expressed HDAC6, confirming that certain antisense oligonucleotides suppress the expression of HDAC6. The control oligo, as expected, did not have any activity of causing the reduction of HDAC6 protein expression.

Also, depending on the reduction in the amount expressed of HDAC6 protein, the acetylation of α-tubulin was increased. This suggested that the deacetylation of α-tubulin is carried out by HDAC6 protein.

Among the above antisense oligonucleotides that exhibited antisense activity, antisense oligonucleotide No. 4 (SEQ ID NO: 6) and No. 5 (SEQ ID NO: 7) in the vicinity of the translation initiation codon ATG, and No. 6 (SEQ ID NO: 8) as the negative control, as well as antisense oligonucleotide No. 16 (SEQ ID NO: 18) located at middle of the coding region and capable of inducing the most potent antisense activity were subjected to a similar experiment as described above by changing the cultivation time (24 hours, 30 hours, 36 hours, and 48 hours) after transfection to cell harvesting.

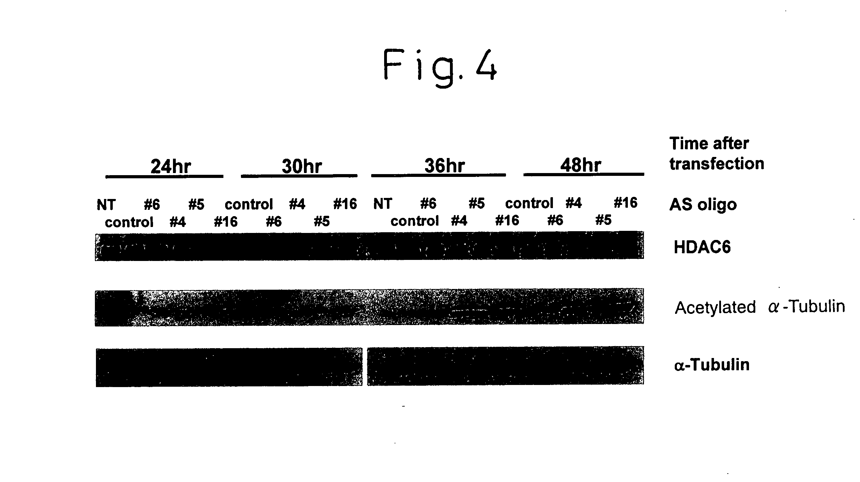

As shown in Figure 4, it was confirmed that for antisense oligonucleotides Nos. 4, 5 and 16, the acetylation of α-tubulin was enhanced at any time. Accordingly, these three antisense oligonucleotides were used in the subsequent experiments.

### Example 5. Changes in microtubule structure due to the reduced expression of HDAC6 protein

HeLa cells were plated on the Lab-TekII Chambered Coverglass (Nunc), and were subjected to:
(1) treatment for 16 hours with 1 µM Paclitaxel (Sigma), a microtubule polymerizing agent;
(2) treatment for 16 hours with 1 µM Trichostatin A (Wako), a HDAC inhibitor; or
(3) the introduction of HDAC6 antisense oligonucleotide No. 4 at a final concentration of 200 nM with OligofectAMINE followed by a 24-hour culture.

Then, Lab-TekII Chambered Coverglass was washed with 0.1 M PIPES (pH 6.9), the cells were fixed in 0.5% glutaraldehyde/0.1 M PIPES (pH 6.9) for 10 minutes, washed three times with PBS, permealized three times with 0.5% Triton X100/PBS for 5 minutes each, washed three times with PBS, treated three times with 2.5 mg/ml sodium borohydride/50% ethanol for 10 minutes each, washed three times with PBS, blocked with 10% normal goat serum/PBS, reacted with a primary antibody (a) anti-acetylated α-tubulin antibody (clone 6-11B-1, Sigma) or (b) anti-α-tubulin antibody (clone DM 1A, Sigma)/PBS for 1 hour, washed three times with 0.1% Tween 20/PBS, reacted with a second antibody Alexa Fluor 488-conjugated anti-mouse IgG (Molecular Probe)/PBS for 30 minutes, and washed three times with 0.1% Tween 20/PBS, and then examined under a conforcal laser scanning microscope (Carl Zeiss).

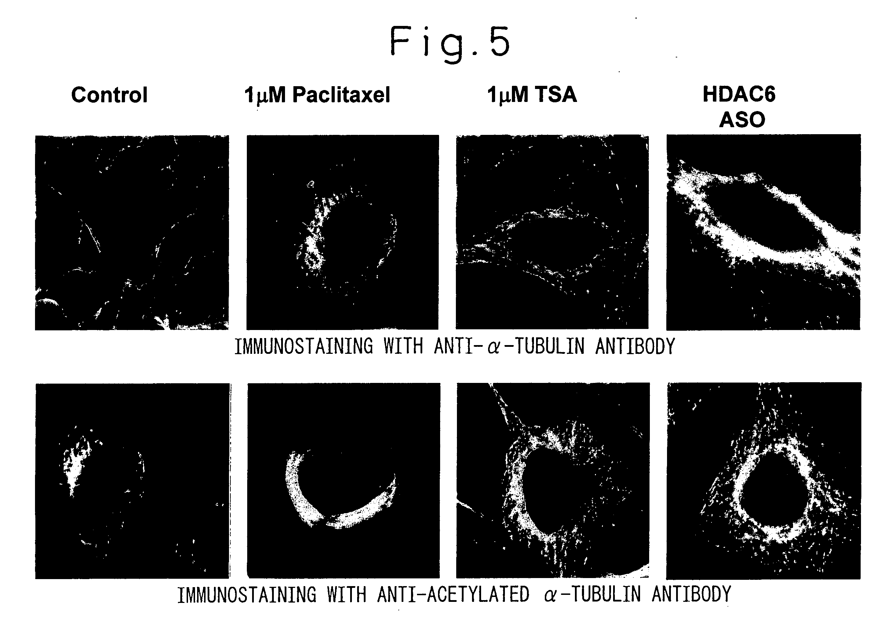

The result is shown in Figure 5. As compared to the untreated control cells, the cells treated with Paclitaxel, a microtubule polymerization-promoter, when stained with anti-α-tubulin antibody, hyperpolymerized microtubules were observed in the periphery of the nucleus. When stained with anti-acetylated α-tubulin antibody, hyperpolymerized microtubules from the centromere were observed.

In the cells treated with Trichostatin A that inhibits the activity of HDAC6 or a HDAC6 antisense oligonucleotide that suppressed the production of HDAC6, unlike treatment with Paclitaxel that is a polymerization promoter of microtubules, images of polymerized microtubules were observed throughout the cytoplasm. It is suggested that in the cells in which α-tubulin deacetylation was inhibited, microtubule stability was enhanced throughout the cytoplasm by an action mechanism different from when cells were treated with Paclitaxel, a polymerization promoter of microtubules.

### Example 6. Induction of apoptosis due to the reduced expression of HDAC6 protein

HeLa cells (1.5 X 10⁵/well) were plated into a 6-well plate with MEM medium containing 10% FCS and 1 X nonessential amino acids. HDAC6 antisense oligonucleotide No. 4, 5, 6 or 16 at the final concentration of 200 nM or the control oligonucleotide was introduced into the cells with OligofectAMINE at 3 µl/well, and the cells were treated for 24 hours, 30 hours, 36 hours, or 48 hours. The cells were detached by trypsin treatment and collected, and stained with Annexin V-PE (Becton Dickinson) according to a protocol attached, and analyzed using the FACS caliber (Becton Dickinson).

Thus, the collected cells were washed with PBS, suspended in 100 µl of the binding buffer (Becton Dickinson), to which suspension 5 µl of Annexin V-PE was added and incubated for 15 minutes, and the binding buffer was added to make a total volume of 500 µl. This was subjected to FACS analysis.

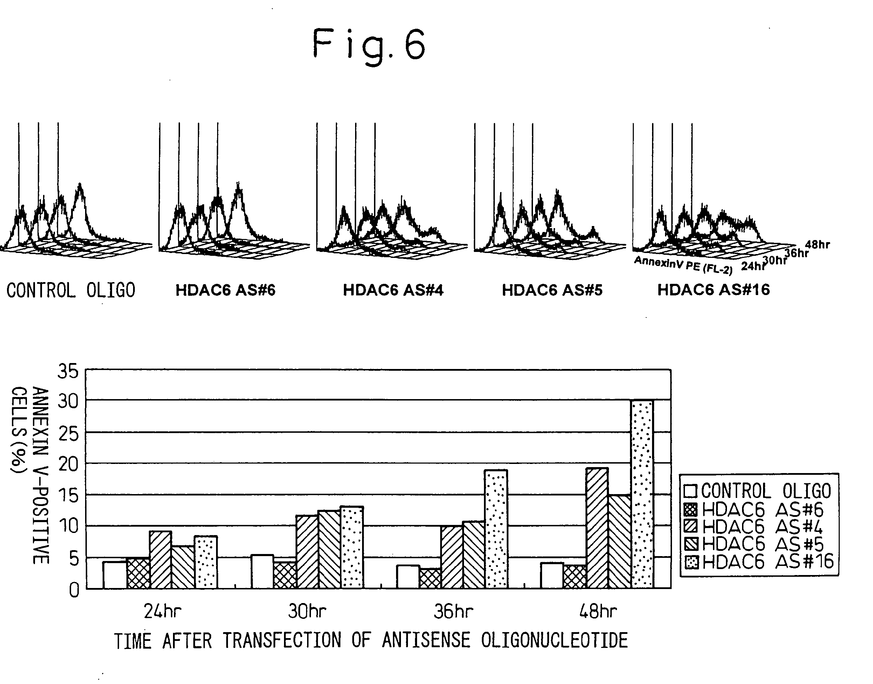

The result is shown in Figure 6. The ratio of Annexin V-positive cells is increased depending on the reduction of the amount expressed of HDAC6 protein. Annexin V is thought to bind phosphatidylserine on the cell membrane which is extracellularly exposed at an early stage of apoptosis. Thus, it is believed that the inhibition of the function of HDAC6 caused apoptosis in the cell.

### Example 7. Induction of depolarization of mitochondrial membrane potential due to the reduced expression of HDAC6 protein

HeLa cells (1.5 X 10⁵/well) were plated into a 6-well plate with MEM medium containing 10% FCS and 1 X nonessential amino acids. HDAC6 antisense oligonucleotide No. 4, 5 or 16 at the final concentration of 200 nM or the control oligonucleotide was introduced into the cells with OligofectAMINE at 3 µl/well, and the cells were treated for 24 hours, 36 hours or 48 hours. Mitochondrial potential sensor JC-1 (Molecular Probes) was added to the medium at the final concentration of 10 µg/ml and the cells were further caltivated at 37°C for 10 minutes. Ten minutes later, the cells were detached by trypsin treatment and collected, and analyzed using the FACS caliber (Becton Dickinson).

When Mitochondrial potential sensor JC-1 is added to the medium, it is rapidly incorporated into mitochondria in the normal cells and the cells exhibit red fluorescence (≅590 nm; FL-2), whereas in the cells in which mitochondrial membrane potential is depolarized, it is not incorporated into mitochondria and is present in the cytoplasm, and the cells exhibit green fluorescence (≅525 nm; FL-1). Therefore, in order to detect the cells in which mitochondrial membrane potential is depolarized, the ratio of green fluorescence-positive cells was graphically shown and used as an index of apoptosis.

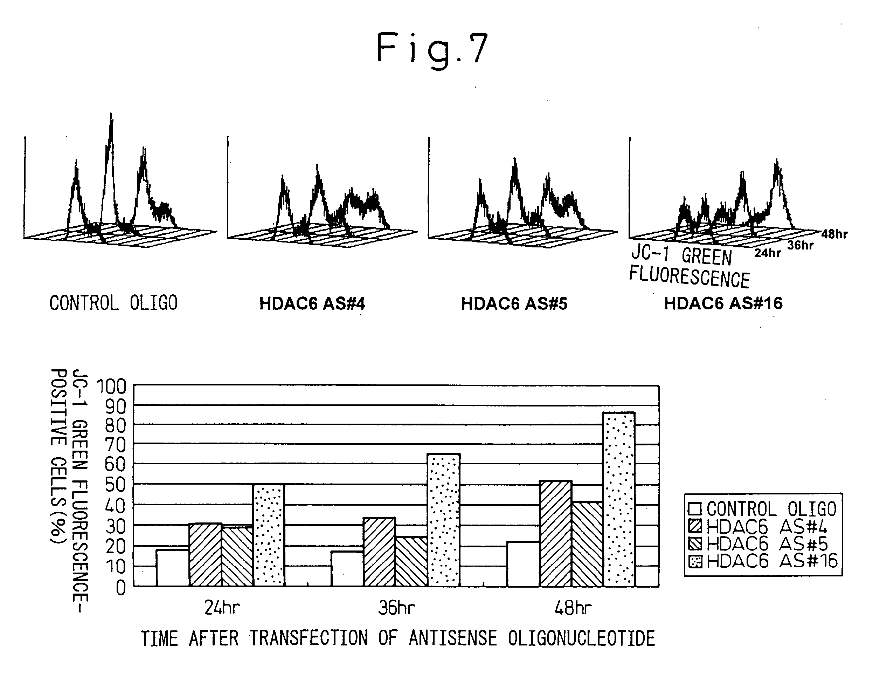

The result is shown in Figure 7. From the result in Figure 3, it can be judged that HDAC6 antisense oligonucleotide has an activity of reducing the amount expressed of HDAC6 protein in the order of No. 16, 4 and
5. From the result in Figure 6, the ratio of cells in which the mitochondrial membrane potential is depolarized is in proportion to the reduction in the amount of expressed HDAC6 protein, and since the inhibition of HDAC6 function causes the enhanced acetylation of α-tubulin in the cell and the depolarization of mitochondrial membrane potential, and therefore it is believed to induce apoptosis.

### Example 8. Suppression of HDAC6 protein expression using phosphorothioate antisense oligonucleotide No. 16 (SEO AD NO: 18) and the resulting enhancement of acetylation of α-tubulin

Using an antisense oligonucleotide No. 16 (in this Example, designated as HDAC ASO) and a control oligonucleotide: CCTCTTACCT CAGTTACAAT (SEQ ID NO: 31) (see, Example 4), suppression of HDAC6 protein expression and resulting enhancement of acetylation of α-tubulin by the antisense oligonucleotide was tested in HepG2 cells (Hepatocellular carcinoma), SW480 cells (Colon adenocarcinoma), LoVo cells (Colon adenocarcinoma) and Calu-1 cells (Lung epidermoid carcinoma).

The synthesized antisense oligonucleotide was dissolved in sterile water, and was introduced into the above-mentioned cells using the OligofectAMINE (Invitrogen) according to an attached protocol. The HepG2 cells at an amount of 5×10⁵ cells/well, SW480 cells at an amount of 4×10⁵ cells/well, LoVo cells at an amount of 4×10⁵ cells/well or Calu-1 cells at an amount of 2×10⁵ cells/well were plated in a 6-well plate with RPMI1640 medium (GIBCO) containing 10% FCS, the final concentration of the antisense oligonucleotide was set at 200 nM, and OligofectANINE was used at 3 µl/well.

Forty-eight hours after the introduction of the antisense oligonucleotide, the cells were detached with trypsin and collected. After the cells were lysed with a SDS-PAGE sample buffer (BPB-free) and protein concentration was determined, the cell lysate at 20 µg/lane was separated by SDS-PAGE, transferred to a PVDF membrane, and immunoblotted with anti-acetylated α-tubulin antibody (clone 6-11B-1, Sigma), anti-α-tubulin antibody (clone DM1A, Sigma) or anti-HDAC6 antibody (H-300, Santa Cruz).

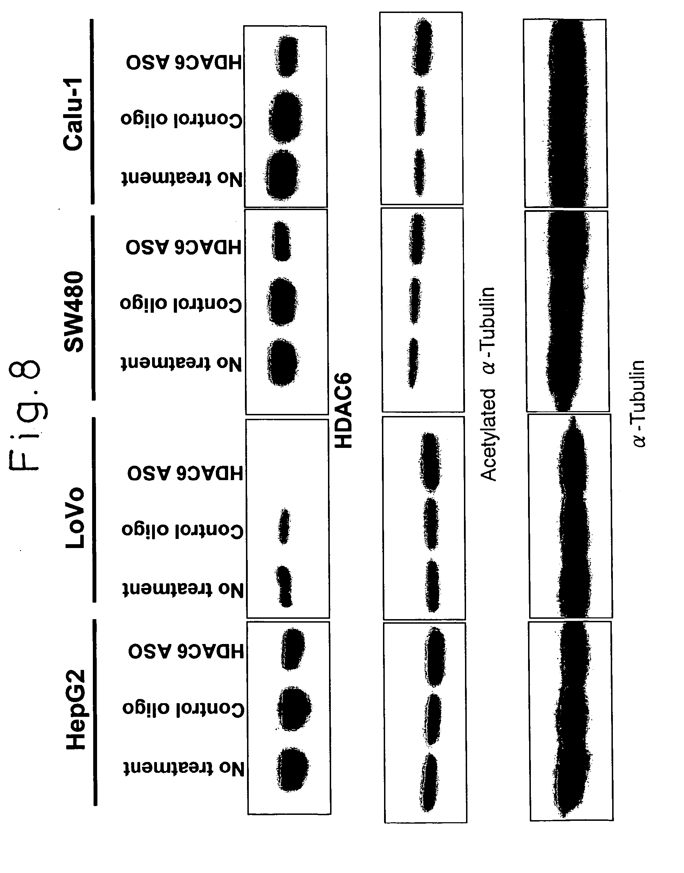

As shown in Figure 8, it was confirmed that the tested antisense oligonucleotide No. 16 (HDAC6 ASO) caused the reduction in the amount of expressed HDAC6. The control antisense oligonucleotide, as expected, did not have any activity of causing the reduction of HDAC6 protein expression. This confirmed that the deacetylation of α-tubulin is carried out by HDAC6 protein.

### Example 9. Induction of depolarization of mitochondrial membrane potential due to the reduced expression of HDAC6 protein by antisense oligonucleotide No. 16

The HepG2 cells at an amount of 5×10⁵ cells/well, SW480 cells at an amount of 4×10⁵ cells/well, LoVo cells at an amount of 4×10⁵ cells/well or Calu-1 cells at an amount of 2×10⁵ cells/well were plated in a 6-well plate with RPMI1640 medium (GIBCO) containing 10% FCS, the HDAC6 antisense oligonucleotide No. 16 or the control anticense oligonucleotide, at the final concentration of 200 nM, was introduced into the cells with OligofectAMINE at 3 µl/well, and the cells were incubated for 48 hours. As a Paclitaxel-treatmrent control, the cells were incubated with 1 µM Paclitaxel for 24 hours. After the incubations, Mitochondrial potential sensor JC-1 (Molecular Probes) was added to the medium at the final concentration of 10 µg/ml and the cells were further incubated at 37°C for 10 minutes. Ten minutes later, the cells were detached by trypsin treatment and collected, and analyzed using the FACS caliber (Becton Dickinson).

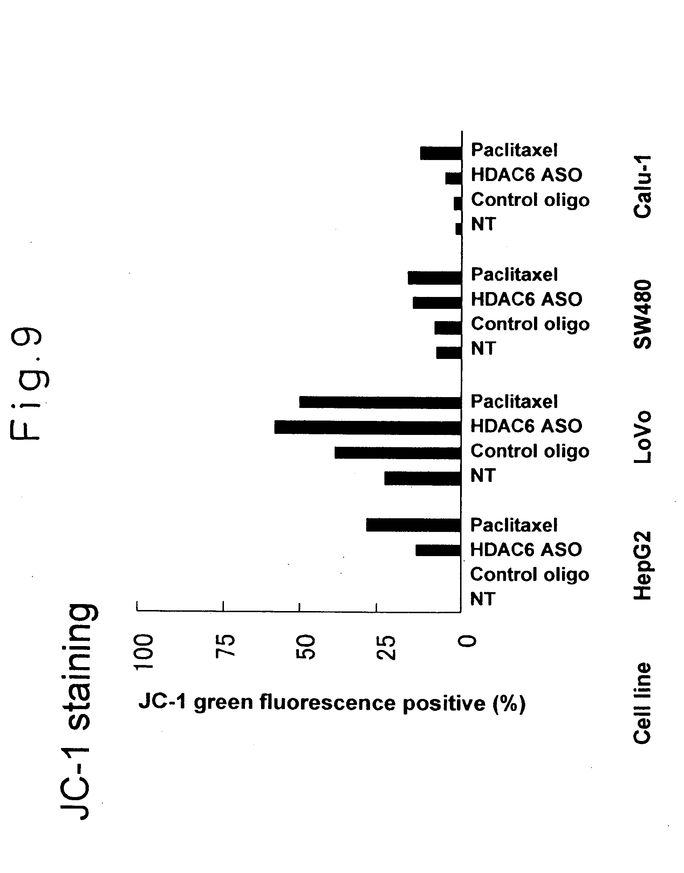

The result is shown in Figure 9. From the result in Figure 8, it can be judged that HDAC6 antisense oligonucleotide No. 16 has an activity of reducing the amount expressed of HDAC6 protein. From the result in Figure 9, the inhibition of HDAC6 function causes the enhanced acetylation of α-tubulin in the cell and the depolarization of mitochondrial membrane potential, and therefore it is believed to induce apoptosis.

### Example 10. Decrease of the number of cells due to apoptosis induced by antisense oligonucleotide No. 16

The HepG2 cells at an amount of 5×10⁵ cells/well, SW480 cells at an amount of 4×10⁵ cells/well, LoVo cells at an amount of 4×10⁵ cells/well or Calu-1 cells at an amount of 2×10⁵ cells/well were plated in a 6-well plate with RPMI1640 medium (GIBCO) containing 10% FCS, the HDAC6 antisense oligonucleotide No. 16 or the control anticense oligonucleotide, at the final concentration of 200 nM, was introduced into the cells with OligofectAMINE at 3 µl/well, and the cells were incubated for 48 hours. As a Paclitaxel-treatmrent control, the cells were incubated with 1 µM Paclitaxel for 24 hours. After the incubations, the cells were collected, and the number of cells was counted by a hemocytometer.

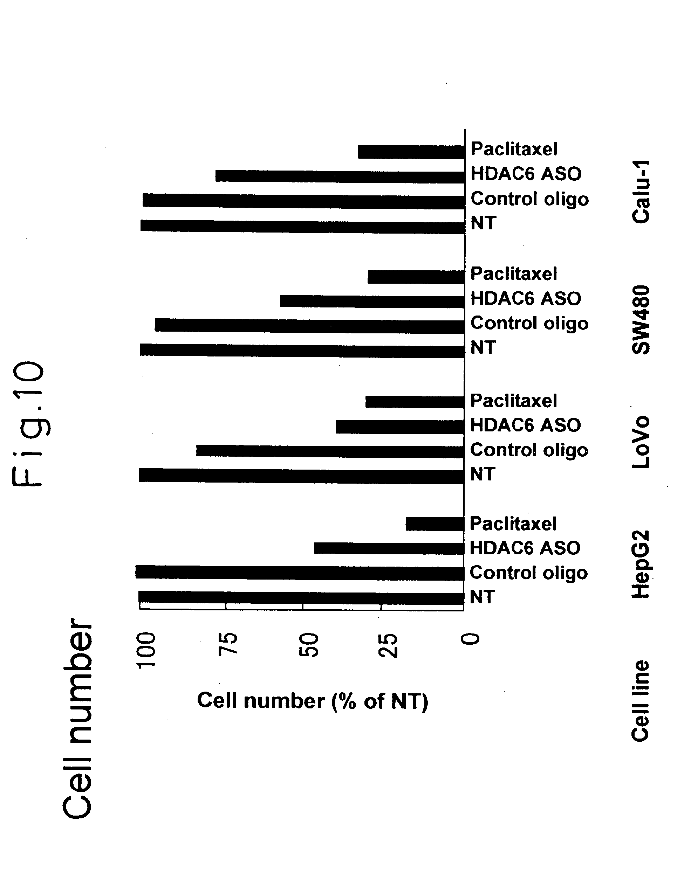

The result is shown in Figure 10. From the result in Figure 10, it can be judged that HDAC6 antisense oligonucleotide has an activity of reducing the amount expressed of HDAC6 protein resulting in the induction of the apoptosis.

Results of Examples 8 to 10 suggest that the death of cells is induced by the apoptosis via mitcondria even in cells other than HeLa cells, on the basis of observations that green fluorescence-positive cells are detected and the number of the cells is decreased, both by inhibiting the formation of HDAC6 protein.

### Example 11. Changes in microtubule structure due to the expression of HDAC6 protein reduced by the antisense oligonucleotide No. 16

HeLa cells were plated on the Lab-TekII Chambered Coverglass (Nunc), and were subjected to:
(1) treatment for 16 hours with 1 µM Paclitaxel (Sigma), a microtubule polymerizing agent;
(2) treatment for 16 hours with 1 µM Trichostatin A (Wako), a HDAC inhibitor; or
(3) the introduction of HDAC6 antisense oligonucleotide No. 16 at a final concentration of 200 nM with OligofectAMINE followed by a 24-hour culture.

Colcemid (a derivative of colchicine) was dissolve in ethanol, and added to the medium to the final concentration of 1 µM, and the cells were incubated for 15 minutes (a time in which the microtubule is almost completely disappears without pre-treatment).

Then, Lab-TekII Chambered Coverglass was washed with 0.1 M PIPES (pH 6.9), the cells were fixed in 0.5% glutaraldehyde/0.1 PIPES (pH 6.9) for 10 minutes, washed three times with PBS, permealized three times with 0.5% Triton X100/PBS for 5 minutes each, washed three times with PBS, treated three times with 2.5 mg/ml sodium borohydride/50% ethanol for 10 minutes each, washed three times with PBS, blocked with 10% normal goat serum/PBS, reacted with a primary antibody rat anti-α-tubulin monoclonal antibody (MAB1864; CHEMICON)/PBS for 1 hour, washed three times with 0.1% Tween 20/PBS, reacted with a second antibody Alexa Fluor 488-conjugated anti-rat IgG (Molecular Probe)/PBS for 30 minutes, and washed three times with 0.1% Tween 20/PBS, and then examined under a conforcal laser scanning microscope (Carl Zeiss).

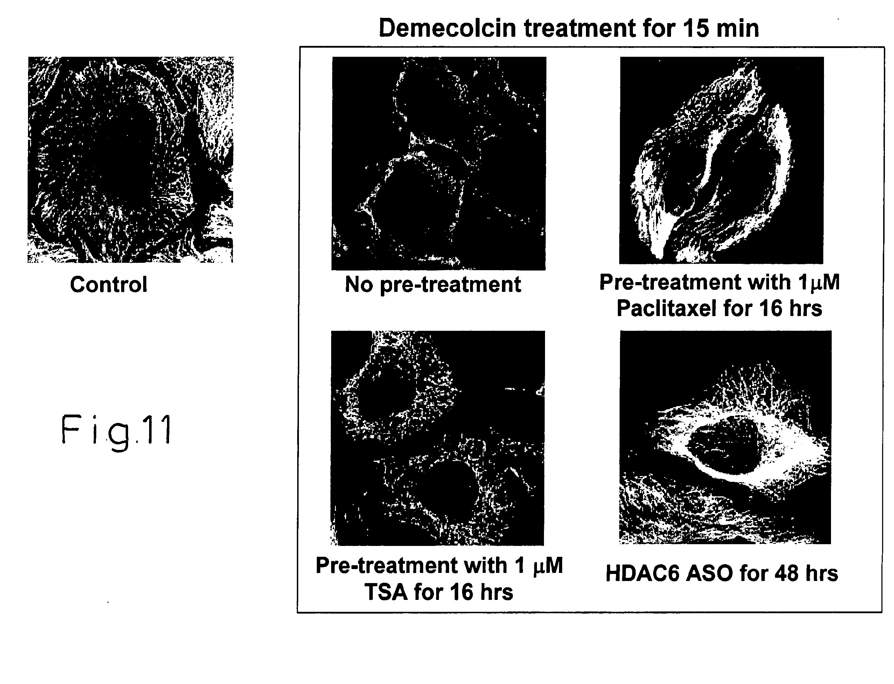

The result is shown in Figure 11. As compared to the cells not pretreated, and treated with Colcemid, wherein the microtuble disappeared, in the cells pretreated with Paclitaxel that is a polymerization promoter of the microtubules, Tricostatin that is an inhibitor of HDAC6, or the HDAC6 antisense oligonucleotide No. 16, the remaining of the polymerized microtubules was observed. It is suggested that inhibition or repression of HDAC6 promotes acetylation of α-tubulin, and the microtubule was stabilized.

### INDUSTRIAL APPLICABILITY

In accordance with the present invention, it was found that the inhibition of histone deacetylase 6 (HDAC6) causes the enhanced acetylation of α-tubulin, resulting in the induction of apoptosis, and hence a substance that inhibits HDAC6 is promising as an anti-cancer agent. Thus, the present invention provides an apoptosis inducer promising as an anti-cancer agent. The present invention also provides a method of screening an apoptosis inducer promising as an anti-cancer agent.

## Claims

1. An apoptosis inducer comprising a substance that inhibits histone deacetylase 6 (HDAC6).

2. An apoptosis inducer according to claim 1
wherein said substance that inhibits histone deacetylase 6 (HDAC6) is a nucleic acid or a peptide.

3. An apoptosis inducer according to claim 2
wherein said nucleic acid is an antisense oligonucleotide or a ribozyme of histone deacetylase 6 (HDAC6).

4. An apoptosis inducer according to claim 2
wherein said nucleic acid is an antisense oligonucleotide of histone deacetylase 6 (HDAC6).

5. An apoptosis inducer according to claim 4
wherein said antisense oligonucleotide has a nucleotide sequence complementary to a sequence comprising an initiation codon ATG of the gene encoding histone deacetylase 6 (HDAC6) or a sequence flanking the initiation codon.

6. An apoptosis inducer according to claim 5
wherein said antisense oligonucleotide has a nucleotide sequence CATAGTTGAG GAGGCTTGGC (SEQ ID No. 3), TCCTGGCCGG TTGAGGTCAT (SEQ ID No. 4), GGTTGAGGTC ATAGTTGAGG (SEQ ID No. 5), CCCTGCCGCG GTTCTTCCAC (SEQ ID No. 6), or GCTGCCTGGT TGTGGTGGAA (SEQ ID No. 7).

7. An apoptosis inducer according to claim 4
wherein said antisense oligonucleotide has a nucleotide sequence CCTTGACCGT GGTGCACATC (SEQ ID No. 10), GTGGTGCACA TCCCAATCTA (SEQ ID No. 11), CCATTACCGT GGTGGACATC (SEQ ID No. 12), GTGGTGGACA TCCCAATCCA (SEQ ID No. 13), ACTGGCCATG TCAGGATTGG (SEQ ID No. 17), or TCCGTAGGGC ATGCCCACTG (SEQ ID No. 18).

8. An anti-cancer agent comprising an apoptosis inducer according to any one of claims 1 to 7 as an active ingredient.

9. A method of screening an apoptosis inducer that inhibits histone deacetylase 6 (HDAC6).

10. A method of screening an apoptosis inducer according to claim 9 said method comprising the steps of:
(1) determining whether or not a test substance inhibits histone deacetylase 6 (HDAC6) by using deacetylation of an acetylated substance that can be a substrate for histone deacetylase 6 (HDAC6) or decrease in the expression of histone deacetylase 6 (HDAC6) as an index; and
(2) further confirming, when inhibition is present, whether it induces apoptosis of the cell in vitro and/or in vivo.
